# EUROPÄISCHE PATENTSCHRIFT

(11) **EP 0 850 044 B1**
(45) Veröffentlichungstag und Bekanntmachung des Hinweises auf die Patenterteilung: **10.04.2002**
(21) Anmeldenummer: 96928644.2
(22) Anmeldetag: 13.09.1996
(51) Int. Cl.: A61K 7/48, C12P 21/06, A23J 3/34, C11D 3/33, C11D 3/37, A61K 7/06

(54) **VERFAHREN ZUR AMINOSÄUREGEWINNUNG AUS PROTEINEN UND VERWENDUNG DERSELBEN AMINOSÄUREHALTIGEN PRODUKTE**
PROCESS FOR OBTAINING AMINO ACIDS FROM PROTEINS AND USE OF THE AMINO-ACID-CONTAINING PRODUCTS OBTAINED
PROCEDE D'OBTENTION D'AMINOACIDES A PARTIR DE PROTEINES, ET UTILISATION DES PRODUITS OBTENUS CONTENANT DES ACIDES AMINES

(30) Priorität: 15.09.1995 DE 19534357; 21.02.1996 DE 19606439
(43) Veröffentlichungstag der Anmeldung: 01.07.1998
(73) Patentinhaber: Zimzik, Henry, 57647 Nistertal (DE)
(72) Erfinder: ZIMZIK, Henry, D-57647 Nistertal (DE); KRAUS, Miroslav, D-35799 Meerenberg (DE)
(74) Vertreter: Blum, Rudolf Emil Ernst
(86) Internationale Anmeldenummer: IB9600939
(87) Internationale Veröffentlichungsnummer: WO9709962

(56) Entgegenhaltungen:
- EP-A- 0 274 939
- EP-A- 0 457 565
- GB-A- 1 308 690
- PATENT ABSTRACTS OF JAPAN vol. 14, no. 548 (C-0785) & JP 02 234642 A (KISSEI PHARMACEUT CO LTD), 17.September 1990,
- DATABASE WPI Week 9428 Derwent Publications Ltd., London, GB; AN 94-230207 XP002024422 "compn. used to treat hypocholesterolaemia" & JP 06 165 655 A (MEIJI MILK PROD CO.LTD) , 14.Juni 1994

## Beschreibung

### Technisches Gebiet

Die vorliegende Erfindung betrifft ein Verfahren zur Anreicherung von Aminosäuren und Oligopeptiden aus Eiweissprodukten, insbesonders aus tierischen Eiweissen.

### Stand der Technik

Aus Milchprodukten abgeleitete Präparate finden heute in Kosmetika bereits breite Anwendung. Die bisher bekannten Verfahren zur Herstellung waschaktiver Substanzen auf der Grundlage von Molke, basieren auf einer Vergärung des in Milchmolken vorhandenen Milchzuckers durch Lactobazillen, die aus Milchzucker Milchsäure zu erzeugen vermögen. Ein solches Verfahren ist z.B. aus DE 28 19 940 bekannt. Weiter beschreibt DE 38 27 833 ein ähnliches Verfahren mit dem Hinweis auf eine kostengünstigere Prozessführung durch Verminderung der Zusätze im Gärverfahren. Weiter beschreibt DE 39 24 559 ein Molken-Behandlungsverfahren, welches insbesondere den Bereich der Frauenheilkunde betrifft. Aus EP 0 357 162 ist ein Verfahren der Milchzuckerspaltung unter Einsatz von Lactobazillen und die Verwendung der daraus entstandenen Produkte bekannt.

Nachteilig wirken sich bei diesen Verfahren die in den Milchmolken vorhandenen Kaseinreste aus, da diese durch Fäulnis Toxine an das Substrat abgeben.

Diesem gravierenden Nachteil kann nur bedingt durch Zusatz stark wirksamer Konservierungsmittel oder gegebenenfalls Eiweissdenaturierungsmittel begegnet werden.

Die so bekannten Herstellungsverfahren verfügen zudem über einen nur ungenügenden Anteil an Milchzucker, um soviel Milchsäure erhalten zu können wie notwendig ist, um die Wasserhärte zu beeinflussen resp. Wasch- und Reinigungsmittel für den Verbraucher zu konzipieren.

WPI/DERWENT 94-230207 offenbart ein Verfahren zur Gewinnung von alpha-Lactalbumin und beta-Lactoglobulin mittels Hydrolyse von Molke durch Protease. Diesem Dokument ist aber kein Abbau der Proteine zu Oligopeptide zu entnehmen.

Die enzymatische Verdauung von isoliertem Eiweiss oder Casein ist EP-A-0 274 939 zu entnehmen. Das Produkt wird in Nahrungsmitteln verwendet.

Patent Abstracts of Japan Vol. 14, no 548 offenbart eine Peptidzusammensetzung, die maximal 10 % Peptide mit einem Molekulargewicht von über 1000 enthält und die - gegebenenfalls nach vorhergehender Behandlung des Ausgangsmaterials mittels Protease - mittels Dipeptidylpeptidase erzeugt wird. Als Protein-Rohmaterial sind spezielle Protein genannt, nicht aber Mischungen, die Proteine zusammen mit anderen Stoffen enthalten.

GB 1 308 690 geht ebenfalls von reinen Proteinen aus, die einer Hydrolyse unterzogen und als Getränkezusatz verwendet werden.

EP 0 457 564 beschreibt das Abbauprodukt von Proteinen resp. deren Abbau bei z.B. pH-Werten von 7 und 8 und die Verwendung der Abbauprodukte in Kosmetika. Es wird ausschliesslich die Verwendung isolierter Proteine beschrieben.

### Darstellung der Erfindung

Ziel der vorliegenden Erfindung war es deshalb, ein Verfahren, sowie ein Produkt bereitzustellen, das von Molke und/oder Quark ausgeht, das eine problemlose Molkeverwertung zulässt, und bei dem die Produkt-Fäulnis weitgehend verhindert wird, so dass auf den Zusatz starker Konservierungsmittel verzichtet werden kann, insbesonders bei gleichzeitiger Verbesserung anderer Produkteigenschaften.

Erfindungsgemäss wird diese Aufgabe durch Bereitstellen eines Verfahrens zur Anreicherung von Aminosäuren und Oligopeptiden gelöst, das dadurch gekennzeichnet ist, dass Molke und/oder Quark in einem durch Sauermolke erzeugten sauren Milieu mit mindestens einer Protease, vorzugsweise mit einem Mikroorganismus behandelt wird. Bevorzugte Ausführungsformen sind in den Unteransprüchen definiert. Weitere Gegenstände der vorliegenden Erfindung sind ein aminosäurehaltiges Produkt, das dadurch gekennzeichnet ist, dass die Aminosäuren als Monoaminosäuren und/ oder Oligopeptide mit max. 9 Aminosäureeinheiten vorliegen sowie die Verwendung der erfindungsgemässen Produkte in Kosmetika, Wasch- und Reinigungsmitteln.

Bei der Spaltung von Eiweissprodukten gemäss der Erfindung wird - wie bereits oben erwähnt - die fermentative Spaltung bevorzugt, da Isolate nicht beabsichtigt sind und die Bildung von Ammoniak und Huminstoffen vermieden werden soll.

Es wird also bewusst auf eine reine Alkaliund/oder Säurespaltung der Eiweissstoffe verzichtet; auch ist die Estermethode nicht Gegenstand der Erfindung.

Die erfindungsgemässe Herstellung eines aminosäurehaltigen Produktes beinhaltet die fermentative Spaltung von Molke und/oder Quark in Verbindung mit Sauermolke, vorzugsweise in Verbindung mit einem Milchserum nach Patent Nr. DE 28 19 940, wobei die natürliche Belassung eines Gemisches bioaktiver, milchsaurer Peptide und Aminosäuren neben Vitaminen und Spurenelementen vorliegt. Erfindungsgemäss wird der Proteinabbau mittels Proteasen durchgeführt. Vorzugsweise wird nicht reine Protease verwendet, sondern proteasereiche Mikroorganismen, wie Enterobacteriaceae/ Proteae. Werden anstelle von proteasehaltigen Mikroorganismen reine Proteasen eingesetzt, so ist das ganze Verfahren weniger gut geregelt, und es können bekannte Proteaseinhibitoren notwendig werden, um unkontrollierten resp. zu starken Abbau zu verhindern.

Süss- oder Sauermolken enthalten mehr oder weniger prozentuale Anteile an Eiweiss (Casein) und sind deshalb ein bevorzugtes Ausgangsmaterial für das erfindungsgemässe Verfahren. Casein ist ein bekanntes, im Handel erhältliches Protein, das aus Magermilch durch Säureoder Enzymfällung erhalten wird, vergleiche Kirk-Othmer, Enzyklopädie der chemischen Technik, 1949, Band 3, Seiten 225-237; und die Encyclopaedia of Polymer Science and Technology, 1965, Interscience Publishers, Band 2, Seiten 859-871. Casein hat in verschiedenen Industriezweigen Verwendung gefunden. Die Aminosäurebilanz der nach den bekannten Verfahren genutzten Milchmolken wiesen aus, dass die darin enthaltenen Eiweissbestandteile nicht allein von Milchsäure erzeugenden Mikroorganismen bis zu Aminosäuren gespaltet werden können, sondern es erfolgt lediglich ein Abbau des Milchzuckers der bekannten Gärungsmolken zu Milchsäure und es ist die Milchsäure, die eine Vorstufe der Eiweissspaltung bewirkt-, vergl. Pschyrembel Klinisches Wörterbuch, 255.Auflage, S. 1068-1069. Mit den Milchsäure erzeugenden Verfahren des Stands der Technik werden die Proteine maximal bis zur Stufe von Peptonen, d.h. bis zu Oligopeptiden mit minimal 10 Aminosäureeinheiten, abgebaut. Ein Produkt mit so langen Aminosäureketten ist immer noch fäulnisanfällig. Ferner sind Proteine und Peptone zu gross, als dass die darin enthaltenen Aminosäuren durch den Organismus, auf dessen Haut sie appliziert werden, voll genutzt werden könnten.

Durch das vorliegende Verfahren werden Proteine und Peptone weiter aufgespalten, bis zur Stufe von Oligopeptiden mit maximal 9 Aminosäureeinheiten resp. Monoaminosäuren. Beim erfindungsgemässen Abbau von Casein ist ein Compound S-haltiger, basischer, heterozyklischer, aromatischer Aminosäuren in Verbindung mit einem milchsauren Medium mit Vitamin A + B und Spurenelementen der folgenden Zusammensetzung zu beobachten:
Asparaginsäure
Asparagin
Alanin
Arginin
Cystin
Glutaminsäure
Glutamin
Histidin
Isoleucin
Leucin
Methionin
Phenylalanin
Prolin
Serin
Threonin
Tyrosin
Valin

Für den Menschen als essentielle Aminosäuren werden bezeichnet: Isoleucin, Leucin, Lysin, Methionin, Phenylalanin, Threonin, Tryptophan, Valin, Histidin und Tyrosin; wobei die Aminosäuren Histidin und Tyrosin nur für den Säugling als essentiell zu bezeichnen sind.

Die Vorstufe zur Gewinnung von Aminosäuren wird vorzugsweise dadurch erreicht, dass die Aminosäurequelle angesäuert wird. Für ein erfindungsgemässes Verfahren, bei dem der Ausgangsstoff Molke ist, sieht ein bevorzugtes Verfahren wie folgt aus:

Die Vorstufe zur Gewinnung von Aminosäuren wird vorzugsweise dadurch erreicht, dass dem Casein mittels bekannter Sauermolken das Kalzium entzogen wird und somit das Gesamtcasein der Molken für eine hydrolytische Spaltung zu Aminosäuren durch proteolytische Enzyme erleichtert wird. Der Eiweissgehalt und damit auch der Aminosäuregehalt im Endprodukt kann durch Zugabe von Quark zur Sauermolke wesentlich erhöht werden. Geeignet ist beispielsweise eine Mischung aus 25 % Quark und 75 % Sauermolke. Höhere Anteile an Quark in bezug auf das Milchserum heben die Aminosäurebilanz an, geringere Anteile senken sie ab. Ein Quarkanteil von ca. 25 % gibt sehr gute Resultate. Entsprechend behandelte Eiweissquellen ergeben ein Produkt, das kaum zu Eiweissfäule neigt, und in dem die Aminosäuren gut zugänglich sind.

Die im erfindungsgemässe Verfahren verwendeten Enzyme sind bekanntlich Proteine, die als Katalysatoren in lebenden Organismen vorkommen und chemische Reaktionen auslösen können, wobei ein Enzym jeweils nur eine für ein Substrat mögliche Reaktion zu katalysieren vermag (Gesetz der Wirkungsspezifität).

Der enzymatische Abbau der in saurem Medium, beispielsweise Sauermolken resp. mit Quark angereicherten Sauermolken, vorliegenden Proteine erfolgt vorzugsweise mikrobiologisch, beispielsweise mit Bacterium caseini und/oder Enterobacteriaceae/Protease (Proteae).

Es wurde gefunden, dass das bevorzugte biologisch aktive System in Verbindung mit Caseinspaltprodukten aus Molke resp. Molke/Quark einen überraschenden Effekt aufweist, der auf Synergismen des Gesamtkomplexes Sauermolke-Aminosäuren beruht.

Weiter hat sich gezeigt, dass das erfindungsgemässe Milchserum-Aminosäure-Compound eine wesentliche und notwendige Bereicherung im Bereich der Kosmetik darstellt und obendrein eine problemlose Molkeverwertung zulässt.

Erfindungsgemäss hergestellte Produkte sind hervorragend geeignet als Bestandteile kosmetischer und/oder medizinischer Präparate und/oder Wasch- und/oder Reinigungsmittel.

Der Amphotere-Charakter des erfindungsgemässen Milchserum-Protein-Gemisches macht es besonders zur Verbesserung tensidhaltiger Reinigungsmittel geeignet.

Das nach dem erfindungsgemässen Verfahren hergestellte Gemisch kann direkt, oder verstärkt durch einen Tensidzusatz, als saures Reinigungsprodukt oder als Zusatz zu Formulierungen wie z.B. Spülmitteln, Allzweckreinigern und/oder Waschmitteln für die Industrie und den Haushalt verwendet werden. Die so entstandenen milieugerechten waschaktiven Formulierungen sind sowohl den biologischen Bedürfnissen des Menschen als auch denen seiner Umwelt besonders gut angepasst. So hat sich gezeigt, dass die Verfahrensprodukte keinerlei allergische Erscheinungen verursachen, im Gegenteil, die so hergestellten Produkte beweisen ein verbessertes Verhalten zum natürlichen Hautmilieu.

Bei der Verwendung der erwähnten kommerziellen Tenside, beispielsweise NPEO und/oder LAS, hat sich gezeigt, dass deren Fischtoxizität in Gegenwart des erfindungsgemäss hergestellten Produkts erheblich geschwächt wird. Die Verfahrensprodukte sind darüberhinaus auch vollständig biologisch abbaubar.

Das erfindungsgemäss hergestellte Produkt besitzt ein gut kontrolliertes biologisches Substanzverhalten gegenüber dem Menschen und seiner Umwelt.

Der pH-Wert des erfindungsgemässen Gemisches liegt bei 3,5 ± 0,5 und kann je nach Anforderung der Weiterverarbeitung entsprechend eingestellt werden.

In Verbindung mit oberflächenaktiven Substanzen wie z.B. Fettalkoholsulfaten, Fettalkoholethersulfaten, Fettalkoholpolyglykolethern, Oelen und Fetten, kann das erfindungsgemässe Produkt auch als Körperreinigungsmittel in Form von Waschlotionen, Dusch- und Schaumbädern eingesetzt werden.

Das erfindungsgemässe Produkt ist aber auch zur Verbesserung hautpflegender Kosmetika und als Zusatz zu medizinischen Präparaten geeignet.

Die erfindungsgemäss hergestellte Zusammensetzung ist beispielsweise auch als Antischuppenshampoo geeignet. Gegenwärtig verwendete Antischuppenshampoos enthalten kolloidalen Schwefel mit Anteilen von Polythionsäuren. Dies gilt zwar als eine gute Wirkstoffkombination gegen Kopfschuppen und vermehrten Haarausfall, wird aber von Dermathologen skeptisch beurteilt.

Es wurde nun festgestellt, dass das erfindungsgemässe Gemisch gute Eigenschaften gegen Kopfschuppen und vermehrten Haarausfall aufweist. Diese positive Wirkung tritt insbesonders in Verbindung mit Salicylsäure auf, beispielsweise Salicylsäure aus salicylsäurehaltigen Pflanzenextrakten resp. in Form solcher Extrakte. Ein solches Gemisch kommt im Resorptionsvermögen ätherischen Oelen fast gleich. Es konnte gezeigt werden, dass in Verbindung mit einer erfindungsgemässen Aminosäure-Oligonukleotidzusammensetzung zur Erreichung der Keratolyse von Kopfhautschuppen eine zirka 40 mal schwächere Konzentration an Salicylsäure nötig ist, als beispielsweise bei der Verwendung einer üblichen Salicyl-Vaseline-Salbe. Ferner hat sich gezeigt, dass durch das erfindungsgemässe Produkt die Versorgung der Haarbalg-Anhangsgebilde mit Aminosäuren als Nährsubstrat verbessert werden kann.

Kondensationsprodukte des erfindungsgemässen Gemisches mit Fett- und/oder Oelsäuren stellen einen ausgesprochen blanden Komplex als Grundlage für kosmetische Cremen und/oder medizinische Pasten dar, wobei der ausgesprochen hohe Anteil an natürlichen Aminosäuren speziell günstig ist.

Zur Verringerung der Transportkosten vom Hersteller zum Weiterverarbeiter kann das erfindungsgemässe Produkt, ohne Schädigung seiner Wirkstoffe, mittels Gefrier- und/oder Vakuumtrocknung erheblich bezüglich Gewicht und Volumen des Ausgangsproduktes reduziert werden.

In der Folge wird das erfindungsgemässe Verfahren anhand von Beispielen näher erläutert. Diese sollen in keiner Weise als den Umfang der Erfindung einschränkend betrachtet werden.

### Präparation 1 (Herstellung von Sauermolke nach DE 28 19 940)

Frische Molke wird im Gärbehälter bis zur Gärmarke aufgefüllt. Diese wird anschliessend mit vorher vorbereiteten Döderlein-Stäbchen geimpft (1000 ml auf 1000 Liter Molke).

Der Garbehälter wird darauf mit dem Gäranschluss versehen und die Molke während ca. 140-150 Std. (ca. 6 Tage) bei Temperatur 20-30°C gären gelassen. Die Molke erreicht ca. pH = 3.

Die vergorene Molke kann für die Lagerung konserviert werden. Die Lagerung soll nur in vollen und hermetisch verschlossenen Behältern erfolgen. Die Haltbarkeit ist begrenzt auf ca. 2 Monate (ohne Zusatz von Stabilisierungsmittel).

Der so vorbereiteten Molke können bis zu 30 % Proteine, z.B. in Form von Quark zugeführt werden.

### Vorbereitung von Döderlein' schen Paracasei/ Döderlein (Lactobacillus/Acidophilus):

Frischer Molke werden ca. 2-5 % Milchzucker zugegeben und gelieferte Döderlein-Stäbchen (mind. 0,1 - 0,2 mm³/100 l Molke). Nach 24 Std. Gärung bei Temperatur 30-35°C ist dieses Gärgemisch zur Verwendung bereit.

Reinkulturen des Lactobacillus Acidophilus können von staatlichen oder privaten Instituten bezogen werden, z.B. von DSM GmbH, D-38124 Braunschweig oder von den Bakteriologischen Instituten der Städte Essen, Koblenz und Frankfurt.

### Beispiel 1

In Nährlösung suspendierte in vitro Zellen (50 ml/100 l) von einer Protease (Enterobacteriaceae/-Proteae, Bezug von den obengenannten Instituten) werden in das Nährmedium Milchserum (Sauermolke, hergestellt nach Präparation 1) inokuliert und ohne weitere Zusätze während 24 Stunden bei 20-30°C, vorzugsweise ca. 27°C, kultiviert. Nach 24 Stunden Kultivierungszeit werden 50 ml der Suspension zu 150 Liter Sauermolke gegeben. Das in der Molke befindliche Restcasein ist nach ca. 7 Tagen zu Aminosäuren und Oligopeptiden von max. 9 Aminosäureeinheiten abgebaut. Das so gewonnene Milchserumprodukt ist transparent und sensorisch ein einheitliches Produkt und wurde als geruchlich oder geschmacklich ohne störende Nebenprodukt festgestellt.

### Beispiel 2

Das Inokulieren der proteasereichen Zellen erfolgte wie in Beispiel 1 beschrieben. Nach 24 Stunden Kultivierungszeit wurden 50 ml der Suspension zu einer Mischung aus 75 Litern Sauermolke und 25 kg Quark gegeben. Das vorhandene Casein ist nach ca. 3-10 Tagen zu Aminosäuren, Di-, Tri- und Oligopeptiden von max. 9 Aminosäureeinheiten abgebaut. Ein spezielles, nach diesem Verfahren erhaltenes Produkt im Vergleich mit einem Produkt nach DE 28 19 940 zeigt die in der untenstehenden Tabelle aufgeführte Zusammensetzung:

**Tabelle**

| Aminosäure | Prod. gemäss Beispiel 2 [g/100 g Probe] | Prod. gemäss DE 28 19 940 [g/100 g Probe] |
|---|---|---|
| Asparaginsäure + Asparagin | 77,4 | 2,81 |
| Threonin | 57, 12 | 2,86 |
| Serin | 50,80 | 1,67 |
| Glutamin + Glutaminsäure | 184,5 | 3,11 |
| Glycin | 25,60 | 9,77 |
| Alanin | 41,78 | 4,92 |
| Valin | 77,96 | 8,98 |
| Isoleucin | 64,05 | 7,01 |
| Leucin | 95,73 | 3,80 |
| Tyrosin | 28,65 | 4,33 |
| Phenylalanin | 45,72 | 8,08 |
| Lysin | 78,01 | 3,70 |
| Arginin | 31,08 | 5,81 |
| Prolin | 128,3 | 6,55 |
| Cystin | 3,61 | - |
| Methionin | 20,73 | - |
| Histidin | 19,92 | - |

## Patentansprüche

1. Verfahren zur Anreicherung von Aminosäuren und Oligopeptiden, bei dem eine Eiweissquelle in einem sauren Milieu mit mindestens einer Protease behandelt wird, **dadurch gekennzeichnet, dass** die Eiweissquelle Molke und/oder Quark ist, dass das saure Milieu durch Sauermolke erzeugt wird und dass die Aufspaltung der Proteine bis zur Stufe von Oligopeptiden mit maximal 9 Aminosäuren erfolgt.

2. Verfahren gemäss Anspruch 1, **dadurch gekennzeichnet, dass** die mindestens eine Protease als Mikroorganismus vorliegt.

3. Verfahren gemäss Anspruch 2, **dadurch gekennzeichnet, dass** der Mikroorganismus ein Mikroorganismus der Gattung Enterobacteriaceae/Protease ist.

4. Verfahren gemäss Anspruch 2 oder 3, **dadurch gekennzeichnet, dass** die Protease enthalten in suspendierten in vitro Zellen vorliegt, dass diese Zellen in ein Nährmedium inokuliert und zur Bildung eines Kulturmediums kultiviert werden.

5. Verfahren gemäss Anspruch 4, **dadurch gekennzeichnet, dass** das Kulturmedium mit weiterem Eiweiss, in Form von Molke und/oder Quark als zusätzliche Eiweissquelle versetzt wird.

6. Verfahren gemäss Anspruch 4 oder 5, **dadurch gekennzeichnet, dass** das Nährmedium Sauermolke ist.

7. Aminosäurehaltiges Produkt, **dadurch gekennzeichnet, dass** die Aminosäuren in Form von Monoaminosäuren und/oder Oligopeptiden mit max. 9 Aminosäureeinheiten vorliegen und dass das Produkt gemäss dem Verfahren nach einem der Ansprüche 1 bis 6 herstellbar ist.

8. Verwendung des aminosäurehaltigen Produkts gemäss Anspruch 7 zur Herstellung von kosmetischen Mitteln.

9. Verwendung des aminosäurehaltigen Produkts gemäss Anspruch 7 zur Herstellung von Wasch- und/ oder Reinigungsmitteln.

10. Medizinisches Präparat, **dadurch gekennzeichnet, dass** es ein aminosäurehaltiges Produkt gemäss Anspruch 7 enthält.

11. Medizinisches Präparat gemäss Anspruch 10, **dadurch gekennzeichnet, dass** es ein Schuppenshampoo ist.

12. Medizinisches Präparat gemäss Anspruch 11, **dadurch gekennzeichnet, dass** es zusätzlich Salicylsäure und/oder ein Salicylsäurederivat enthält.

13. Aminosäurehaltiges Produkt gemäss Anspruch 7 als Mittel zur Behandlung von Schuppen und vermehrtem Haarausfall.

## Claims

1. Process for the enrichment of amino acids and oligopeptides in which a protein source is treated in an acidic medium with at least one protease, **characterised in that** the protein source is whey and/or curd, **in that** the acidic medium is generated by sour whey, and **in that** the degradation of the proteins is performed to the level of oligopeptides with at most 9 amino acids.

2. Process according to claim 1, **characterised in that** the at least one protease is present as micro-organism.

3. Process according to claim 2, **characterised in that** the micro-organism is a micro-organism of the variety Enterobacteriaceae/Proteae.

4. Process according to claim 2 or 3, **characterised in that** the proteases are present comprised in suspended in vitro cells, **in that** said cells are inoculated in a nutritive medium and cultivated in order to form a culture medium.

5. Process according to claim 4, **characterised in that** the culture medium is provided with further protein in the form of whey and/or curd as additional protein source.

6. Process according to claim 4 or 5, **characterised in that** the nutritive medium is sour whey.

7. Amino acids comprising product, **characterised in that** the amino acids are present in the form of mono amino acids and/or oligopeptides with at most 9 amino acid units, and **in that** said product is produceable according to the process of anyone of claims 1 to 6.

8. Use of an amino acids comprising product according to claim 7 for the preparation of cosmetic preparations.

9. Use of an amino acids comprising product according to claim 7 for the preparation of detergents and/or cleansers.

10. Medical preparation, **characterised in that** it comprises an amino acids comprising product according to claim 7.

11. Medical preparation according to claim 10, **characterised in that** it is a dandruff shampoo.

12. Medical preparation according to claim 11, **characterised in that** it furthermore comprises salicylic acid and/or a salicylic acid derivative.

13. Amino acids comprising product according to claim 7 as agent for the treatment of dandruff and enhanced loss of hair.

## Revendications

1. Procédé d'enrichissement en acides aminés et oligopeptides, dans lequel une source protéique est traitée en milieu acide avec au moins une protéase, **caractérisé en ce que** la source protéique est du petit-lait ou du fromage blanc, **en ce que** le milieu acide est produit par du lait caillé et **en ce que** le clivage des protéines est réalisé jusqu'à l'étape d'oligopeptides ayant au maximum 9 acides aminés.

2. Procédé suivant la revendication 1, **caractérisé en ce que** la protéase, au moins une, est présente sous la forme d'un microorganisme.

3. Procédé suivant la revendication 2, **caractérisé en ce que** le microorganisme est un microorganisme de l'ordre des entérobactériacées/ protéases.

4. Procédé suivant la revendication 2 ou 3, **caractérisé en ce que** la protéase est présente dans les cellules en suspension in vitro, **en ce que** ces cellules sont inoculées à un milieu nutritif et **en ce qu'**elles sont cultivées pour former un milieu de culture.

5. Procédé suivant la revendication 4, **caractérisé en ce que** l'on ajoute au milieu de culture, d'autres protéines, sous forme de petit-lait et/ou fromage blanc comme source supplémentaire de protéines.

6. Procédé suivant la revendication 4 ou 5, **caractérisé en ce que** le milieu nutritif est du lait caillé.

7. Produit contenant des acides aminés, **caractérisé en ce que** les acides aminés sont présents sous la forme de mono(acides aminés) et/ou d'oligopeptides ayant au maximum 9 unités acide aminé et **en ce que** le produit peut être préparé par le procédé suivant l'une quelconque des revendications 1 à 6.

8. Utilisation du produit contenant des acides aminés suivant la revendication 7, pour préparer des produits cosmétiques.

9. Utilisation du produit contenant des acides aminés suivant la revendication 7, pour préparer des agents de lavage et de nettoyage.

10. Préparation médicale, **caractérisée en ce qu'**elle contient un produit contenant des acides aminés suivant la revendication 7.

11. Préparation médicale suivant la revendication 10, **caractérisée en ce qu'**il s'agit d'un shampooing anti-pelliculaire.

12. Préparation médicale suivant la revendication 11, **caractérisée en ce qu'**elle contient en outre, de l'acide salicylique et/ou un dérivé de l'acide salicylique.

13. Produit contenant des acides aminés suivant la revendication 7, comme agent pour traiter les pellicules et la chute des cheveux.
